(19) Europäisches Patentamt
European Patent Office
Office européen des brevets

(11) **EP 3 984 461 B1**

(12) **FASCICULE DE BREVET EUROPEEN**

(45) Date de publication et mention de la délivrance du brevet:
**12.06.2024 Bulletin 2024/24**

(21) Numéro de dépôt: **21201226.4**

(22) Date de dépôt: **06.10.2021**

(51) Classification Internationale des Brevets (IPC):
**A61B 6/00** *(2024.01)* **H04N 5/32** *(2023.01)*
**H05G 1/42** *(2006.01)*

(52) Classification Coopérative des Brevets (CPC):
**A61B 6/542; H05G 1/44;** H04N 5/32; H04N 25/76

(54) **PROCEDE DE CONTROLE D'EXPOSITION EN TEMPS REEL D'UNE DOSE DE RAYONS X**

VERFAHREN ZUR ECHTZEIT-ÜBERWACHUNG DER EXPOSITION EINER RÖNTGENSTRAHLENDOSIS

METHOD FOR MONITORING THE REAL-TIME EXPOSURE OF A DOSE OF X-RAYS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.10.2020 FR 2010567**

(43) Date de publication de la demande:
**20.04.2022 Bulletin 2022/16**

(73) Titulaire: **Trixell**
**38430 Moirans (FR)**

(72) Inventeurs:
• **WIRTH, Thibaut**
**38430 MOIRANS (FR)**
• **CHARLES, Benoit**
**38430 MOIRANS (FR)**

(74) Mandataire: **Atout PI Laplace**
**Immeuble Up On**
**25 Boulevard Romain Rolland**
**CS 40072**
**75685 Paris Cedex 14 (FR)**

(56) Documents cités:
**EP-A2- 1 001 665 US-A- 5 194 736**

EP 3 984 461 B1

**Description**

L'invention se situe dans le domaine technique de la radiographie avec un capteur plan numérique et plus particulièrement des dispositifs de contrôle en temps réel du niveau d'exposition.

Les paramètres d'exposition (tension, courant, distances source-patient-détecteur) doivent être ajustés pour minimiser la dose reçue par le patient tout en garantissant une qualité d'image optimale pour le radiologue. Les paramètres optimaux d'exposition dépendent de la corpulence du patient, du type d'examen demandé et des caractéristiques de sensibilité de l'imageur de rayons X (film argentique, plaque ERLM, capteur plan numérique, etc...). La détermination de ces paramètres est en général assurée par le logiciel du système, en fonction des indications sur le contexte de l'examen (corpulence du patient, type de cliché demandé) fournies par le manipulateur radio. Si ces paramètres sont mal définis, soit du fait d'indications erronées ou imprécises, soit du fait d'un mauvais étalonnage du système, il existe un risque significatif de surexposition ou de sous-exposition du cliché. Ces risques impliquent le risque d'une surexposition du patient aux rayons X, soit directement, soit parce que l'image doit être refaite.

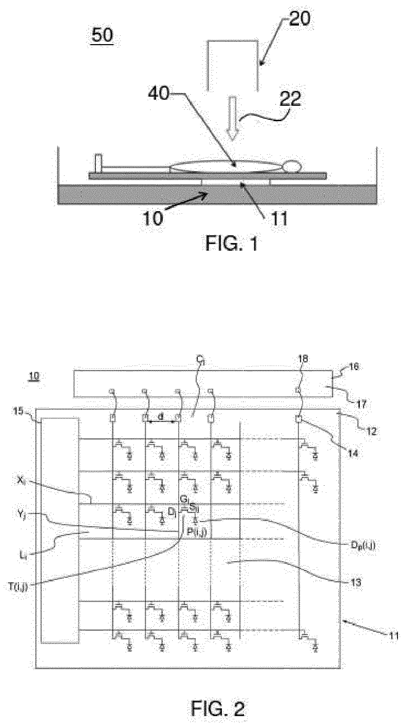

La figure 1 représente schématiquement un ensemble radiologique 50 traditionnel. Un ensemble de radiologie 50 est constitué de deux éléments : un tube générateur 20 d'un faisceau de rayons X 22 et un capteur plan 11 d'images radiographiques. L'ensemble est destiné à réaliser principalement des images radiographiques de patients en milieu hospitalier. Un patient, dont on souhaite faire une radiographie d'une zone d'intérêt 40, est placé entre le tube générateur 20 d'un faisceau de rayons X 22 et le capteur plan 11. Les deux éléments doivent donc être bien positionnés l'un par rapport à l'autre, de façon à ce que tous les rayons X émis par le tube générateur 20 du faisceau de rayons X soient captés par le capteur plan 11. On parle alors de bon alignement entre les deux éléments. L'alignement doit se faire avant que les rayons X ne soient émis par le tube générateur 20 du faisceau de rayons X. Le but est d'éviter de sur-irradier le patient avec des rayons X arrivant en dehors du capteur. Il existe plusieurs façons connues de procéder à l'alignement de deux éléments.

Outre le bon alignement du faisceau de rayons X avec le capteur plan, il est important de contrôler les paramètres d'exposition afin de s'assurer qu'une dose de rayons X suffisante a été transmise afin de garantir une bonne image mais que le patient n'a pas été sur-irradié.

Jusqu'à présent, le problème de quantification de la dose d'exposition est résolu en pratique par l'une ou l'autre des solutions suivantes :

- Dispositif AEC (abréviation de l'acronyme anglo-saxon Automatic Exposure Control) externe du type « chambre d'ionisation » ou « capteur état solide ». Il s'agit d'un bloc fonctionnel de contrôle automatique de dose (par exemple chambre d'ionisation), reposant sur un bloc matériel « capteur de rayons X + amplificateur de courant » séparé de l'imageur et connecté au bloc de contrôle du générateur de rayons X. Le capteur est placé devant l'imageur et absorbe une très faible fraction des rayons X pour ne pas perturber l'image. Le capteur est compartimenté par zones d'intérêt (en général 3 ou 5 zones) et peut fournir un courant électrique pour chaque zone à l'amplificateur. L'amplificateur est relié d'un côté au capteur et de l'autre côté au bloc de contrôle du générateur de rayons X. La fonction de l'amplificateur est de fournir au générateur un signal sans retard correspondant au niveau total d'exposition actualisé. Ce signal permet au bloc de contrôle du générateur d'interrompre l'émission de rayons X (la calibration des niveaux d'arrêt se fait lors de l'installation du système).

- Dispositif AEC (Automatic Exposure Control) interne, généralement par exploitation du signal reçu sur une partie de l'image. Deux types principaux de solutions existent. Dans un premier type de solution, la matrice de pixels est modifiée physiquement de telle sorte qu'un signal électrique spécifiquement dédié à la mesure du signal soit acheminé vers un convertisseur charge-tension d'abord puis analogique-numérique ensuite. Dans un second type de solution (divulgué par exemple dans le document JP5481 053), la matrice n'est a priori pas modifiée, mais il n'est pas fait mention du problème de signal parasite provenant de tous les pixels illuminés qui ne sont pas dans la zone d'intérêt définie pour la régulation de l'exposition.

- Dispositif dit « Preshot » réalisant une exposition préliminaire avec une très faible dose. Un « pré-cliché » du patient est réalisé à très faible dose juste avant le « vrai cliché ». Aucun signal en temps réel n'est fourni au système, mais les paramètres finaux sont déterminés et mis en place automatiquement par le système à l'aide d'un algorithme d'analyse du pré-cliché.

La solution AEC externe a trois principaux défauts. Elle intègre en général un capteur absorbant les rayons X en amont de l'imageur, ce qui engendre un surcroît de dose sur le patient de l'ordre de quelques pourcents. Dans certains cas, le capteur externe peut être en aval de l'imageur, ce qui fausse l'information sur la qualité du faisceau vu par l'imageur et demande donc une calibration poussée. En outre, cette solution AEC s'applique uniquement aux systèmes fixes du fait de son encombrement : le capteur est à fixer sur l'imageur, il faut un câble pour acheminer le signal vers l'amplificateur. Elle ne s'applique donc pas aux systèmes de radiographie mobiles, ni à la prise d'image sur un détecteur portable hors de son logement dédié. Enfin, elle est financièrement coûteuse pour l'intégrateur du système : pour une salle typique avec table et potence, il faut deux blocs AEC, dont chacun coûte 500 euros au moins.

La solution AEC interne présente les inconvénients suivants. Une modification de la matrice de pixels est nécessaire, ce qui ne permet pas d'implémenter la solution sur des produits déjà existants. Bien que proposée dans la littérature, elle est difficile à réaliser du fait du problème technique du couplage capacitif entre les pixels éclairés et la colonne de lecture du signal AEC.

La solution dite Preshot a également deux principaux défauts. Elle nécessite de calibrer parfaitement le système, c'est-à-dire d'établir une corrélation entre les paramètres pour une faible dose (pré-cliché) et les paramètres pour une dose normale (cliché). Cette corrélation doit également prendre en compte le contexte de l'examen (corpulence du patient, type de cliché demandé). Aussi, elle suppose un délai entre le pré-cliché et le cliché qui peut détériorer le flux d'application : temps d'attente, effet de mouvement du patient, etc... Le document EP 1001665 A2 divulgue un dispositif AEC qui utilise la capacité de couplage parasite comme source de signaux pour le système de commande d'exposition et qui estime la capacité de couplage avec un facteur multiplicatif.

L'invention vise à pallier tout ou partie des problèmes cités plus haut en proposant un procédé de contrôle d'exposition en temps réel d'une dose de rayons X permettant d'indiquer au système, en temps réel, le niveau de gris dans l'image en cours de formation dans l'imageur (capteur plan numérique, ci-après désigné par détecteur), pour une zone d'intérêt de l'image prédéterminée afin de garantir le bon niveau d'exposition du patient. Ce procédé est basé sur l'utilisation directe de la dalle du détecteur sans modification, pour analyser en temps réel le niveau de signal dans les zones d'intérêt de l'image. Par ailleurs, le procédé selon l'invention propose une méthode algorithmique pour résoudre le problème important du couplage entre le signal d'intérêt (aussi appelé signal utile) et le signal parasite provenant notamment des zones illuminées sans collimation. Enfin, le procédé selon l'invention peut se décliner sans connexion filaire, ce qui permet son implémentation sur un système de radiographie mobile ou en mode cassette portable dans une salle de radiographie.

A cet effet, l'invention a pour objet un procédé de contrôle d'exposition en temps réel d'une dose de rayons X émise par un tube générateur d'un faisceau de rayons X et reçue par un détecteur comprenant un capteur plan, le tube générateur comprenant un bloc de contrôle du tube générateur configuré pour contrôler une dose de rayons X émise par le tube générateur, ledit capteur plan comprenant

a. un ensemble de pixels organisé en matrice selon des lignes et des colonnes et configuré de façon à générer des signaux en fonction de la dose de rayons X frappant le détecteur ;
b. un circuit configuré pour déterminer un signal utile à partir des signaux issus d'au moins une des lignes ;
c. un module de transmission du signal utile au bloc de contrôle du tube générateur ;

ledit procédé de contrôle étant caractérisé en ce qu'il comprend les étapes suivantes :

- exposition du capteur plan à une dose de rayons X émise par le tube générateur d'un faisceau de rayons X ;
- lecture répétée d'au moins une des lignes de pixels pendant l'exposition du capteur plan à la dose de rayons X ;
- détermination d'un signal utile et d'un signal parasite à partir des signaux issus de la lecture de la au moins une des lignes ;
- transmission du signal utile au bloc de contrôle du tube générateur.

Avantageusement, le procédé de contrôle d'exposition selon l'invention comprend en outre une étape d'adaptation de la dose de rayons X émise par le tube générateur en fonction du signal utile transmis au bloc de contrôle du tube générateur.

L'étape de lecture répétée d'au moins une des lignes de pixels comprend, pour chaque ligne de la au moins une des lignes, les étapes suivantes :

- lecture de la colonne sans activation de la ligne Li pour obtenir un premier signal An ;
- lecture de la colonne avec activation de la ligne Li pour obtenir un deuxième signal Bn.

L'étape de détermination d'un signal utile et d'un signal parasite comprend les étapes suivantes :

- Estimation d'un facteur multiplicatif (FM') entre le signal utile et le signal parasite ;
- Estimation du signal utile à partir du facteur multiplicatif selon la relation :

$$DS'_n = (A_n + B_n) / (FM' + 1), \text{ et } S'_n = \text{Cumul} (A+B)_n / (FM' + 1).$$

L'étape d'estimation du facteur multiplicatif (FM') entre le signal utile et le signal parasite comprend les étapes suivantes :

- à partir de 3 échantillons successifs de premiers signaux $(A_{n-1}, A_n, A_{n+1}) = (VA)_n$, estimation d'une mesure $A'_n$ du signal parasite au temps du deuxième signal $B_n$ ; (par exemple par interpolation avec le filtre spline suivant : $A'_n =$ $1/16.(-1\ 10\ 7).^t(VA)_n)$
- régression linéaire sur 3 points successifs $\{(x_i,y_i) ; i = 1$ à $3\}$ pour obtenir un coefficient de proportionnalité FP, avec :

  ○

$$\{x_i\} = [Cumul(A+B)_{n-2}, Cumul(A+B)_{n-1}, Cumul(A+B)_n]$$

  ○

$$\{y_i\} = [Cumul(B-A')_{n-2}, Cumul(B-A')_{n-1}, Cumul(B-A')_n ]$$

  ○ $Cumul(A)_n$ étant égal à $A_0+A_1+...+A_n$

- Calcul du facteur multiplicatif avec la relation $FM' =(FP)^{-1} - 1$.

Dans un mode de réalisation, l'étape de transmission du signal utile au bloc de contrôle du tube générateur est réalisée par transmission filaire.

Dans un autre mode de réalisation, l'étape de transmission du signal utile au bloc de contrôle du tube générateur est réalisée par transmission sans fil, préférentiellement par transmission RF.

Un autre aspect concerne aussi un ensemble de radiologie comprenant :

a. un tube générateur d'un faisceau de rayons X, le tube générateur comprenant un bloc de contrôle du tube générateur configuré pour contrôler une dose de rayons X émise par le tube générateur ;
b. un détecteur comprenant un capteur plan, ledit capteur plan comprenant :

   i. un ensemble de pixels (P(i,j)) organisé en matrice selon des lignes (Li) et des colonnes (Cj) et configuré de façon à générer des signaux en fonction de la dose de rayons X frappant le détecteur ;
   ii. un circuit configuré pour déterminer un signal utile à partir des signaux issus d'une partie des lignes (Li) ;
   iii. un module de transmission du signal utile au bloc de contrôle du tube générateur.

Dans un mode de réalisation, le module de transmission du signal utile au bloc de contrôle du tube générateur est un module de transmission filaire.

Dans un autre mode de réalisation, le module de transmission du signal utile au bloc de contrôle du tube générateur est un module de transmission sans fil, préférentiellement RF.

L'invention sera mieux comprise et d'autres avantages apparaîtront à la lecture de la description détaillée d'un mode de réalisation donné à titre d'exemple, description illustrée par le dessin joint dans lequel :

[Fig.1] La figure 1 représente schématiquement un ensemble radiologique traditionnel ;
[Fig.2] La figure 2 représente un détecteur d'images traditionnel ;
[Fig.3] La figure 3 représente un diagramme des étapes du procédé de contrôle d'exposition en temps réel d'une dose de rayons X selon l'invention ;
[Fig.4] La figure 4 représente schématiquement un ensemble radiologique selon l'invention ;
[Fig.5] La figure 5 illustre le principe de positionnement des lignes de la matrice du détecteur dédiées à la lecture ainsi que la lecture répétée selon l'invention ;
[Fig.6] La figure 6 représente les résultats d'une simulation d'une exposition avec une montée rapide en la mise en oeuvre du procédé selon l'invention ;
[Fig.7] La figure 7 représente les résultats d'une simulation d'une exposition avec une montée lente en la mise en oeuvre du procédé selon l'invention.

Sur ces figures, dans un souci de clarté, les échelles ne sont pas respectées. Par ailleurs, les mêmes éléments porteront les mêmes repères dans les différentes figures.

De manière générale, l'invention mentionne un détecteur d'images traditionnel comprenant typiquement un capteur plan comprenant un ensemble de pixels organisé en matrice selon des lignes et des colonnes, des blocs d'adressage de lignes, des blocs de lecture de colonnes, des conducteurs de ligne reliant les lignes de pixels à un bloc d'adressage de lignes, et des conducteurs de colonne reliant les colonnes de pixels à un bloc de lecture de colonnes. Il est à noter que, dans le cadre de la présente demande de brevet, les notions de colonne et de ligne n'ont qu'un sens relatif, une ligne de pixels et une colonne de pixels n'étant autres que des rangées de pixels disposées par exemple, et de manière non-limitative, perpendiculairement l'une à l'autre. Un conducteur de ligne, respectivement de colonne, est défini comme étant orienté parallèlement à une ligne de pixels, respectivement une colonne de pixels.

La figure 1 représente schématiquement un ensemble radiologique 50 traditionnel et a déjà été présentée en introduction.

La figure 2 représente un détecteur d'images 10 traditionnel. Le détecteur d'images 10 comprend un capteur 11 réalisé sur un premier substrat monolithique 12. Le premier substrat monolithique 12 comprend un ensemble de pixels $P(i,j)$ organisé en matrice 13 selon des lignes $L_i$ et des colonnes $C_j$. La matrice 13 peut comporter un nombre quelconque de lignes et de colonnes formant ainsi des pixels $P(i,j)$. La matrice 13 forme une zone géographique sur le premier substrat 12. On note les pixels sous la forme générique $P(i,j)$, où $i$ et $j$ sont des entiers naturels désignant respectivement le rang de la ligne et le rang de la colonne dans la matrice 13. L'ensemble des pixels $P(i,j)$ est configuré de façon à générer des signaux en fonction d'un rayonnement frappant le détecteur 10. Le capteur 11 comprend des conducteurs de colonne $Y_j$, chacun reliant les pixels d'une même colonne $C_j$. Les conducteurs de colonnes $Y_j$ sont destinés à transporter les signaux générés par les pixels $P(i,j)$. De même, le capteur 11 comprend des conducteurs de ligne $X_i$, chacun reliant les pixels d'une même ligne $L_i$. La matrice 13 de pixels $P(i,j)$ comporte des colonnes $C_j$ de rangs pairs et de rangs impairs. De même, la matrice 13 de pixels $P(i,j)$ comporte des lignes $L_i$ de rangs pairs et de rangs impairs. Le capteur 10 comprend des plots de contact 14 situés à la périphérie du premier substrat 12 et en dehors de la matrice 13 de pixels $P(i,j)$. Les plots de contact 14 sont reliés aux conducteurs de colonne $Y_j$. Le détecteur d'images 10 comprend un bloc d'adressage de lignes 15 situé à proximité du premier substrat 12 et relié aux conducteurs de lignes $X_i$. On appelle bloc d'adressage de lignes 15 tout ensemble comportant au moins un bloc d'adressage de lignes. Le bloc 15 peut être intégré au premier substrat 12, comme représenté sur la figure 1, ou bien intégré à un substrat distinct. Le bloc d'adressage de lignes 15 permet d'adresser individuellement chaque ligne de pixels $L_i$. Le détecteur d'images 10 comprend un bloc de lecture de colonnes 16 réalisé sur un second substrat 17 différent du premier substrat 12. Le bloc de lecture de colonnes 16 comprend des points de connexion 18 reliant le bloc de lecture de colonnes 16 aux plots de contact 14. Le bloc de lecture de colonnes 16 permet de lire les signaux générés par les pixels de la ligne sélectionnée par le bloc d'adressage de lignes.

Un pixel $P(i,j)$ comporte une photodiode $Dp(i,j)$ associée à un interrupteur électronique $T(i,j)$. Les photodiodes $Dp(i,j)$ peuvent naturellement être remplacées par tout élément photosensible apte à générer un signal électrique lorsqu'il est soumis à un rayonnement de photons. La structure de pixel représentée sur la figure 2 est volontairement simplifiée et des structures plus complexes peuvent être mises en oeuvre dans le cadre de l'invention.

L'interrupteur $T(i,j)$ formé par un transistor est relié par sa grille $G_i$ au conducteur de ligne $X_i$ de la ligne $i$, par son drain $D_j$ au conducteur de colonne $Y_j$ et par sa source $S_{ij}$ à la cathode de la photodiode $Dp(i,j)$. Les anodes de toutes les photodiodes $Dp(i,j)$ sont reliées à un potentiel commun, par exemple la masse. Le bloc d'adressage de ligne 15 comporte des éléments permettant de générer les signaux à injecter sur les conducteurs de ligne $X_i$ pour piloter l'ouverture et la fermeture des transistors $T(i,j)$. Le bloc de lecture de colonne 16 peut comporter des éléments permettant de traiter les signaux reçus sur les conducteurs de colonne $Y_j$. En particulier, il peut s'agir d'un amplificateur et/ou d'un convertisseur analogique - numérique.

Traditionnellement, le capteur d'images 11 fonctionne de la manière suivante. Lors d'une phase de prise d'image, l'exposition des photodiodes $Dp(i,j)$ à un rayonnement génère des charges électriques au niveau de la source $S_{ij}$. La quantité de charges au niveau de chaque source $S_{ij}$ est fonction de l'intensité du rayonnement reçu par le pixel $P(i,j)$ considéré. La phase de prise d'image est suivie d'une phase de lecture effectuée ligne par ligne. Les signaux injectés sur les différents conducteurs de ligne $X_i$ passent successivement à l'état actif, de sorte que le potentiel de chaque conducteur de colonne $Y_j$ est successivement représentatif de la quantité de charges électriques accumulées dans les différents pixels $P(i,j)$ de la colonne $j$.

Comme expliqué précédemment, la quantification de la dose d'exposition dans l'art antérieur nécessite une modification de la matrice de pixels. Et même si la matrice n'est pas modifiée, l'art antérieur ne propose aucune solution pour éliminer le signal parasite inévitable.

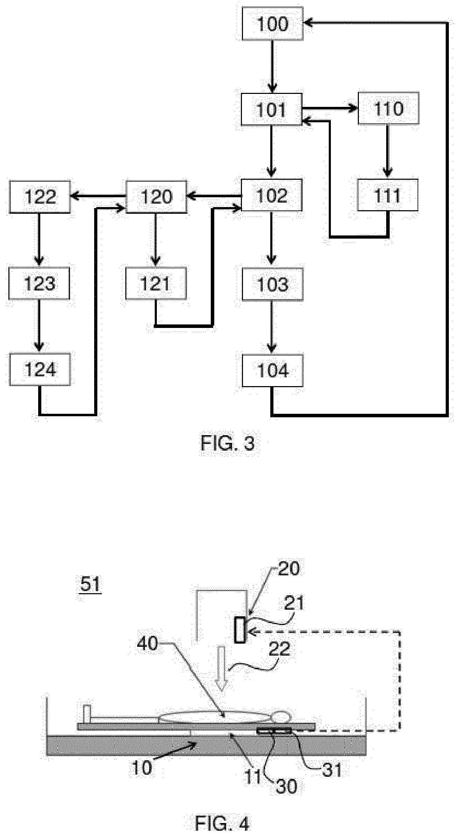

La figure 3 représente un diagramme des étapes du procédé de contrôle d'exposition en temps réel d'une dose de rayons X selon l'invention. Pour une meilleure compréhension des éléments, il peut être fait référence à la figure 2 dans la mesure où la solution proposée ne nécessite pas de modification de la matrice de pixels.

L'invention concerne un procédé de contrôle d'exposition en temps réel d'une dose de rayons X 22 émis par un tube générateur 20 d'un faisceau de rayons X et reçue par un détecteur 10. Le détecteur 10 comprend un capteur plan 11 et le tube générateur 20 comprend un bloc de contrôle 21 du tube générateur 20 configuré pour contrôler une dose de rayons X émise par le tube générateur 20. Le capteur plan 11 comprend :

- un ensemble de pixels P(i,j) organisé en matrice 13 selon des lignes Li et des colonnes Cj et configuré de façon à générer des signaux en fonction de la dose de rayons X 22 frappant le détecteur 10 ;

- un circuit 30 configuré pour déterminer un signal utile à partir des signaux issus d'au moins une des lignes Li ;

- un module de transmission 31 du signal utile au bloc de contrôle du tube générateur 20.

Le circuit 30 est préférentiellement un circuit intégré. Toutefois, d'autres variantes équivalentes peuvent être utilisées. Autrement dit, tout dispositif permettant de déterminer un signal utile à partir des signaux issus d'une des lignes Li convient.

Selon l'invention, le procédé de contrôle comprend les étapes suivantes :

- exposition (étape 100) du capteur plan 11 à une dose de rayons X 22 émise par le tube générateur 20 d'un faisceau de rayons X ;

- lecture répétée (étape 101) d'au moins une des lignes Li de pixels P(i,j) pendant l'exposition du capteur plan 11 à la dose de rayons X 22;

- détermination (étape 102) d'un signal utile et d'un signal parasite à partir des signaux issus de la lecture de la au moins une des lignes Li ;

- transmission (étape 103) du signal utile au bloc de contrôle 21 du tube générateur 20.

L'étape 101 de lecture répétée d'au moins une des lignes Li de pixels P(i,j) de la matrice 13 a lieu pendant l'étape d'exposition 100. Autrement dit, cette étape de lecture a lieu à un moment où, traditionnellement, aucune ligne Li n'est lue dans l'art antérieur. En effet, en mode classique, le détecteur 10 ne fait rien pendant la phase d'exposition.

En outre, on peut noter que l'étape 101 de lecture est effectuée sur une ou plusieurs lignes Li de la matrice de pixels. Ces lignes sont présentes et contribuent au fonctionnement normal du détecteur. En d'autres termes, aucune modification de la matrice de pixels n'est nécessaire pour la mise en oeuvre de cette étape spécifique de lecture de lignes pendant l'exposition aux rayons X.

Afin d'obtenir une étape 101 de lecture la plus rapide possible, il est possible d'ouvrir toutes les lignes prévues à cette étape 101 en même temps, ce qui aura pour effet négatif de confondre toutes les zones d'intérêt en une seule information. Il est également possible de n'activer que la ou les lignes correspondant à une zone d'intérêt prédéfinie.

L'exposition des pixels lors de l'étape 100 d'exposition entraîne la création de charges hors des pixels P(i,j), dans la colonne Cj, car un couplage capacitif relie chaque colonne de la matrice et tous les pixels (transistor et photodiode) rattachés à cette colonne. De ce fait, la lecture d'un pixel en cours d'exposition (étape 100) n'est pas représentative du niveau dans l'image comme le serait une lecture après que l'étape 100 exposition soit terminée : il s'y ajoute un signal parasite des charges créées dans la colonne par couplage. Ce signal parasite peut être important car pour chaque colonne, le couplage provient de tous les pixels illuminés de la colonne. Comme le pixel d'intérêt est en général sous le patient, si beaucoup d'autres pixels sont illuminés sans atténuation du flux direct (faible collimation), le signal parasite peut valoir jusqu'à cent fois le signal utile. Il est donc impératif de prendre en compte ce signal parasite lors de l'étape 102 de détermination des signaux (utile et parasite).

Pour déterminer le signal utile et le signal parasite, l'étape 101 de lecture répétée d'au moins une des lignes Li de pixels comprend, pour chaque ligne Li de la au moins une des lignes Li, les étapes suivantes :

- lecture (étape 110) de la colonne Cj sans activation de la ligne Li pour obtenir un premier signal $A_n$ ;
- lecture (étape 111) de la colonne Cj avec activation de la ligne Li pour obtenir un deuxième signal $B_n$.

On effectue ainsi une double-lecture pour chaque ligne dédiée à la lecture de l'étape 101 : on lit d'abord la colonne sans activer la ligne (étape 110) pour obtenir un échantillon $A_n$ pour la $n^{\text{ième}}$ double-lecture, puis on relit la colonne avec activation de la ligne (étape 111) pour obtenir un échantillon $B_n$ pour la $n^{\text{ième}}$ double-lecture. Cette double-lecture permet de soustraire l'effet parasite. Ces double-lectures sont répétées jusqu'à la fin de la fenêtre XRW (XRW étant l'abréviation du terme anglo-saxon X-ray window pour fenêtre de rayons X, c'est-à-dire la durée pendant laquelle le détecteur est apte à recevoir des photons X et à les convertir en charges électriques avant la phase de lecture). A partir des informations obtenues par les double-lectures $A_n$ et $B_n$, il va être possible d'inférer le niveau de signal réel dans l'image.

La simple soustraction $(B_n-A_n)$ des double-lectures ne permet pas d'obtenir à coup sûr l'augmentation de signal utile $DS_n$, parce que le flux de dose n'est pas constant tout au long de l'exposition (étape 100). Dans la phase initiale d'augmentation du flux de dose, la première lecture $A_{n0}$ non nulle de la colonne a un niveau inférieur à ce qu'il serait si on lisait la colonne à la date de la lecture $B_{n0}$. Autrement dit, la lecture $B_{n0}$ contient plus de signal parasite que $A_{n0}$. La soustraction $B_{n0}-A_{n0}$ contient donc du signal parasite et surévalue le signal utile ajouté $DS_{n0}$. Inversement, en cas de décroissance du flux, par exemple après un maximum dans l'exposition (« overshoot »), le signal $A_{n1}$ est supérieur au signal parasite contenu dans $B_{n1}$ et donc la soustraction $B_{n1}-A_{n1}$ sous-évalue la variation de signal utile $DS_{n1}$, voire donne un résultat négatif.

Pour résoudre ce problème, la solution proposée par l'invention prend pour hypothèse que la durée d'exposition minimale se situe légèrement en-dessous d'une durée brève, à titre illustratif de l'ordre de 1 ms, par exemple 0.8 ms, et que la contrainte selon laquelle un signal électrique d'arrêt doit atteindre le bloc de contrôle du générateur peu de temps après, par exemple moins de 0.1 ms après, l'atteinte effective d'un certain niveau de signal dans l'imageur (contrainte CP) peut être levée en-dessous de ce temps. Ceci signifie qu'on se donne un délai tampon de 0.8 ms pour l'analyse au départ de l'exposition, et que passé ce délai, on respecte à nouveau la contrainte (CP). En termes d'analyse de danger, cela signifie que la responsabilité de ne pas surexposer le patient, qui incombe toujours au système de radiographie, repose avant tout sur un choix de paramètres non aberrants pendant ce premier délai de 0.8 ms.

Dans la suite, l'hypothèse principale (HP), selon laquelle l'algorithme dispose d'un délai de 0.8 ms à partir du début de l'exposition avant de devoir respecter définitivement la contrainte (CP), est supposée valide.

On va alors utiliser ce délai (HP) pour estimer, de façon robuste, un facteur multiplicatif FM' entre le signal utile et le signal parasite pour une exposition en cours. En effet, ce facteur reste constant tout au long de l'exposition, quel que soit le profil temporel du flux de dose, car il est entièrement déterminé par la configuration d'exposition relative entre les pixels d'intérêt d'une colonne et le reste des pixels de la colonne (pour chaque colonne). Si plusieurs colonnes sont jointes pour augmenter la quantité de signal à chaque lecture, le facteur multiplicatif à appliquer à cet ensemble de colonnes restera également constant.

Pour ce faire, l'étape 102 de détermination d'un signal utile et d'un signal parasite comprend les étapes suivantes :

- Estimation (étape 120) d'un facteur multiplicatif (FM') entre le signal utile et le signal parasite ;
- Estimation (étape 121) du signal utile à partir du facteur multiplicatif selon la relation :

$$DS'_n = (A_n+B_n) / (FM'+1), \text{ et } S'_n= \text{Cumul }(A+B)_n / (FM' +1).$$

Plus précisément, l'étape 120 d'estimation du facteur multiplicatif FM' entre le signal utile et le signal parasite comprend les étapes suivantes :

- à partir de 3 échantillons successifs de premiers signaux $(A_{n-1}, A_n, A_{n+1}) = (VA)_n$, estimation (étape 122) d'une mesure $A'_n$ du signal parasite au temps du deuxième signal $B_n$ ;
- régression linéaire (étape 123) sur 3 points successifs $\{(x_i,y_i) ; i = 1 \text{ à } 3\}$ pour obtenir un coefficient de proportionnalité FP, avec :

  ○

$$\{x_i\} = [\text{Cumul}(A+B)_{n-2}, \text{Cumul}(A+B)_{n-1}, \text{Cumul}(A+B)_n]$$

  ○

$$\{y_i\} = [\text{Cumul}(B-A')_{n-2}, \text{Cumul}(B-A')_{n-1}, \text{Cumul}(B-A')_n ]$$

  ○ $\text{Cumul}(A)_n$ étant égal à $A_0+A_1+...+A_n$

- Calcul (étape 124) du facteur multiplicatif avec la relation $FM' =(FP)^{-1} - 1$.

L'étape 122 peut être réalisée par exemple par interpolation avec le filtre spline suivant : $A'_n = 1/16.(-1\ 10\ 7).^t(VA)_n)$

Cette estimation est bonne si la forme de l'impulsion est bien approximée par un polynôme entre les 3 points mesurés, ce qui est courant. D'autres filtres ont été testés, comme par exemple la moyenne arithmétique (0 0.5 0.5).$^t$(A).

L'estimation A'$_n$ correspond à ce que serait la mesure du signal parasite au temps de B$_n$.

L'étape 123 de régression linéaire est justifiée par le fait que la quantité Cumul(A+B)$_n$ est en principe proportionnelle au niveau de signal accumulé au temps de B$_n$, avec un facteur multiplicatif égal à FM'+1, qui correspond à l'accumulation des signaux parasites (FM'*DS$_n$) et des signaux utiles (1*DS$_n$). Le signal parasite ajouté au temps de B$_n$ est uniquement estimé par A'$_n$, ce qui, en cas de variation brusque de l'impulsion, peut conduire à un décalage du signal reconstitué par rapport au signal réel.

L'idée de la régression sur 3 points permet de rattraper ce décalage, pour peu que survienne une période moins « chahutée » de l'impulsion, qui dure le temps de quelques double-lectures (sans pour autant que le flux n'ait à rester constant).

Le coefficient de proportionnalité FP donné par la régression linéaire ci-dessus permet donc de donner une estimation FM' du facteur multiplicatif par la relation : FM' + 1 = (FP)$^{-1}$, soit FM' =(FP)$^{-1}$ - 1

Si le coefficient de corrélation CC de la régression est jugé suffisamment proche de 1 (par exemple |1-CC| < 10$^{-5}$) alors on peut considérer que FM est estimé de façon robuste par FM'.

Si le coefficient de corrélation CC n'est pas suffisamment proche de 1, alors on réitère les étapes 122 et 123 en passant de n à n+1, tout en vérifiant que le délai donné par l'hypothèse principale (HP) n'est pas dépassé.

Une fois le facteur multiplicatif estimé avec une confiance suffisante, on est capable de donner une estimation DS'$_n$ du niveau du signal utile DS$_n$ ajouté à chaque temps de B$_n$ par la formule : DS'$_n$ = (A$_n$+B$_n$) / (FM'+1)

Et donc on obtient aussi : S'$_n$= Cumul (A+B)$_n$ / (FM' +1)

Ce signal utile est transmis au bloc de contrôle du tube générateur.

Enfin, le procédé de contrôle d'exposition selon l'invention comprend avantageusement une étape 104 d'adaptation de la dose de rayons X 22 émise par le tube générateur 20 en fonction du signal utile transmis au bloc de contrôle 21 du tube générateur 20. Cela garantit donc que le patient reçoit la bonne dose de rayons X pour permettre l'obtention d'une image de qualité, sans surexposition inutile.

Grâce à l'invention, une solution algorithmique de correction du couplage capacitif entre pixels et colonne est donnée.

Ces étapes ont montré de bons résultats sur simulation (Figures 2 et 3). La situation est plutôt favorable pour une impulsion courte, car le flux de dose est alors élevé et chaque échantillon a un bon rapport signal/bruit. C'est une bonne nouvelle puisque les situations critiques du point de vue du client sont plutôt celles à impulsion courte.

Par contre, pour des impulsions longues (dizaines de ms), le flux de dose peut être faible et le rapport signal/bruit se détériore pour chaque lecture. Pour pallier ce problème, outre le cumul sur plusieurs colonnes (effet de moyenne), on propose que l'information de durée de fenêtre X (XRW), passée au détecteur lors de la demande de trame, soit utilisée pour définir un pas de temps de lecture plus long, tout en maintenant l'objectif d'avoir une erreur de signal estimé de moins de 10%.

L'étape 103 de transmission du signal utile au bloc de contrôle du tube générateur peut être réalisée par transmission filaire, ou par transmission sans fil, préférentiellement par transmission RF. De manière générale, il faut une transmission rapide ayant un temps de latence minimal et bien maîtrisé. Pour cette raison, les liaisons de type Wi-Fi et Bluetooth, bien qu'applicables dans le cadre de l'invention, sont moins bien adaptées.

Avantageusement, la transmission de l'information est réalisée en temps réel. L'information du niveau de signal au temps de B$_n$ obtenue à l'étape 102 doit être transmise au bloc de contrôle 21 du tube générateur 20 avec un retard inférieur à 0.1 ms par rapport au temps de B$_n$. L'algorithme permettant de réaliser l'étape 102 étant implémenté dans un circuit intégré de type FPGA, et son principe ne nécessitant pas beaucoup de calculs, la plus grande partie de cette contrainte sur le retard se reporte sur l'étape 103 de transmission de l'information depuis le circuit intégré vers le bloc de contrôle. Le cas d'une liaison filaire ne pose pas de problème particulier. Le cas d'une liaison sans fil, de type RF, est nettement plus difficile.

Dans le cas où le retard lié à la liaison RF est supérieure à 0.1 ms, on peut envisager une étape d'extrapolation de l'information fournie à l'étape 102, à condition que celle-ci soit horodatée et que les éléments émetteur (module de transmission) et récepteur (bloc de contrôle) soient synchronisés au préalable.

La figure 4 représente schématiquement un ensemble radiologique 51 selon l'invention. L'ensemble de radiologie 51 comprend :

- un tube générateur 20 d'un faisceau de rayons X, le tube générateur 20 comprenant un bloc de contrôle 21 du tube générateur 20 configuré pour contrôler une dose de rayons X 22 émise par le tube générateur 20 ;

- un détecteur 10 comprenant un capteur plan 11, ledit capteur plan 11 comprenant :

   o un ensemble de pixels P(i,j) organisé en matrice 13 selon des lignes Li et des colonnes Cj (similairement au détecteur présenté à la figure 2) et configuré de façon à générer des signaux en fonction de la dose de rayons X 22 frappant le détecteur 10 ;

    o un circuit 30 configuré pour déterminer un signal utile à partir des signaux issus d'une partie des lignes Li.

    o un module de transmission 31 du signal utile au bloc de contrôle 21 du tube générateur 20.

Le circuit 30 peut être un circuit intégré. Le circuit intégré peut être tout circuit apte à réaliser des calculs. Il peut s'agir à titre d'exemple et de manière non-limitative d'un FPGA (abréviation de « Field Programmable Gate Arrays » signifiant réseaux logiques programmables). Le circuit intégré réalise les étapes d'analyse des données des lignes dédiées pour sortir l'information de niveau de signal à un moment donné. L'information est acheminée vers le bloc de contrôle du générateur, soit directement par une liaison filaire, soit par l'intermédiaire d'une liaison hertzienne (option RF).

Le module de transmission 31 du signal utile au bloc de contrôle 21 du tube générateur 20 peut être un module de transmission filaire, ou un module de transmission sans fil, préférentiellement RF. Il transmet l'information de signal à un moment donné vers le module récepteur du tube générateur. Le module récepteur reçoit l'information de niveau de signal à un moment donné, la convertit en signal électrique et la fournit au bloc de contrôle du générateur X.

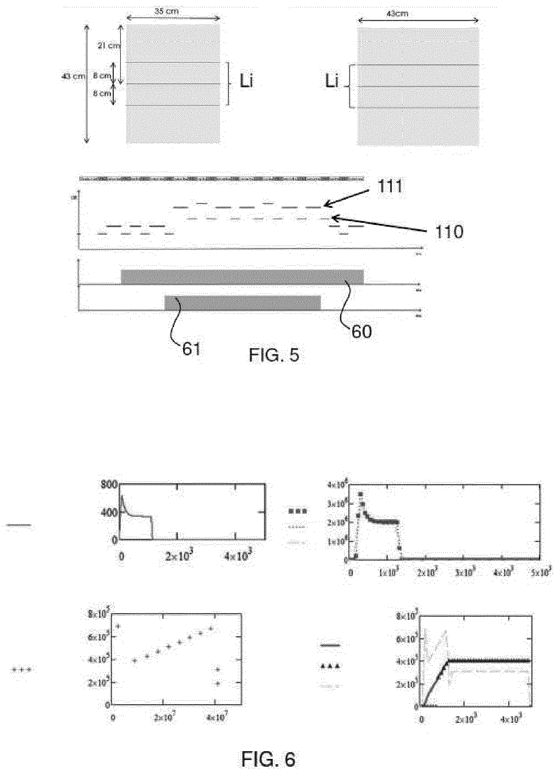

La figure 5 illustre le principe de positionnement des lignes de la matrice du détecteur dédiées à la lecture ainsi que la lecture répétée selon l'invention. Seules les lignes intervenant dans l'étape 101 de lecture répétée sont représentées (partie supérieure de la figure). Il s'agit de lignes existantes, déjà présentes dans la matrice du détecteur. Les mesures sont données à titre indicatif pour montrer des exemples possibles de réalisation. Sur cette figure, trois lignes sont utilisées pour l'étape 101. Il pourrait n'y en avoir qu'une seule, ou bien deux, ou encore plus. Ces lignes sont lues de manière continue aussi vite que possible pendant la fenêtre de rayons X 60 (la référence 61 représente la fenêtre d'émission de rayons X). Pour avoir une valeur représentative, au moins la lecture de la colonne sans activer la ligne (étape 110) et la lecture de la colonne avec activation de la ligne sont obligatoires. Cela signifie que pendant la fenêtre de rayon X, alternativement l'étape 110 est réalisée puis l'étape 111 (c'est-à-dire les colonnes avec les lignes activées sont lues).

La figure 6 représente les résultats d'une simulation d'une exposition avec une montée rapide en la mise en oeuvre du procédé selon l'invention.

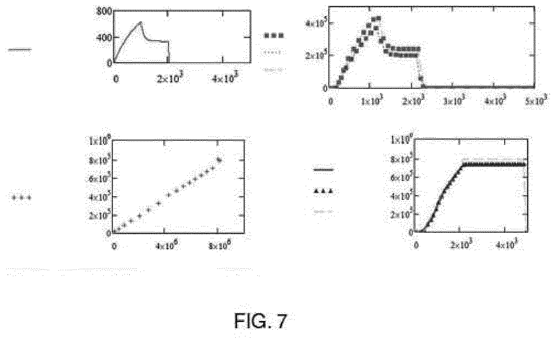

La figure 7 représente les résultats d'une simulation d'une exposition avec une montée lente en la mise en oeuvre du procédé selon l'invention.

Pour ces deux figures, nous voyons en haut à droite la forme d'impulsion simulée. En bas à gauche est représenté le graphe de $Cumul(B-A')_n$ en fonction de $Cumul(A+B)_n$. En bas à droite, nous voyons la vérification du fonctionnement de l'estimation en fonction du temps écoulé : signal utile vrai (représenté en trait plein), signal utile estimé par $Cumul(A+B)_n/(FM'+1)$ (représenté par les triangles), qui est non nul dès que CC est suffisamment proche de 1, et ce que serait l'estimation $Cumul(B-A')_n$ (représenté en pointillés).

Sur la figure 6, les échantillons $A_n$ et $B_n$ sont simulés avec un facteur multiplicatif FM=100. On voit que la régression linéaire peut fonctionner dès le 2ème triplet de points.

Sur la figure 7, les échantillons $A_n$ et $B_n$ sont simulés avec un facteur multiplicatif FM=10.

Ces deux figures démontrent l'efficacité de la solution proposée pour le problème de couplage capacitif.

L'invention propose un procédé de contrôle de l'exposition en temps réel avec les avantages de pouvoir le mettre en oeuvre sans modification physique ni adaptation spécifique de la matrice de photodiodes (lecture rapide des lignes dédiées existantes de la dalle pendant l'exposition). L'analyse du niveau de signal dans les zones d'intérêt de l'image se fait en temps réel. L'étape 102 permet de résoudre le problème important du couplage entre le signal d'intérêt et le signal parasite provenant notamment des zones illuminées sans collimation.

Enfin, ce procédé est compatible avec une transmission sans connexion filaire, étendant les possibilités d'utilisation à la radiographie mobile ou en mode cassette portable dans une salle de radiographie.

La solution proposée donne un niveau de signal en temps réel. Elle consiste à utiliser directement la dalle du détecteur sans modification, pour analyser en temps réel le niveau de signal dans les zones d'intérêt de l'image. Par ailleurs, la solution proposée décrit une méthode algorithmique pour résoudre le problème important du couplage entre le signal d'intérêt et le signal parasite provenant notamment des zones illuminées sans collimation. Enfin, la solution proposée peut se décliner sans connexion filaire, ce qui permet l'implémentation sur un système de radiographie mobile ou en mode cassette portable dans une salle de radiographie.

## Revendications

**1.** Procédé de contrôle d'exposition en temps réel d'une dose de rayons X émis par un tube générateur (20) d'un faisceau de rayons X et reçue par un détecteur (10) comprenant un capteur plan (11), le tube générateur (20) comprenant un bloc de contrôle (21) du tube générateur (20) configuré pour contrôler une dose de rayons X émise par le tube générateur (20), ledit capteur plan (11) comprenant

a. un ensemble de pixels (P(i,j)) organisé en matrice (13) selon des lignes (Li) et des colonnes (Cj) et configuré de façon à générer des signaux en fonction de la dose de rayons X (22) frappant le détecteur (10) ;
b. un circuit (30) configuré pour déterminer un signal utile à partir des signaux issus d'au moins une des lignes (Li) ;
c. un module de transmission (31) du signal utile au bloc de contrôle du tube générateur (20) ;
ledit procédé de contrôle comprant les étapes suivantes :

- exposition (100) du capteur plan (11) à une dose de rayons X émise par le tube générateur (20) d'un faisceau de rayons X ;
- lecture répétée (101) d'au moins une des lignes (Li) de pixels (P(i,j)) pendant l'exposition du capteur plan (11) à la dose de rayons X ;
- détermination (102) d'un signal utile et d'un signal parasite à partir des signaux issus de la lecture de la au moins une des lignes (Li) ;
- transmission (103) du signal utile au bloc de contrôle (21) du tube générateur (20) ;
ledit procédé de contrôle étant **caractérisé en ce que** l'étape (101) de lecture répétée d'au moins une des lignes (Li) de pixels comprend, pour chaque ligne (Li) de la au moins une des lignes (Li), les étapes suivantes :

- lecture (110) de la colonne (Cj) sans activation de la ligne Li pour obtenir un premier signal An ;
- lecture (111) de la colonne (Cj) avec activation de la ligne Li pour obtenir un deuxième signal Bn ;

**en ce que** l'étape (102) de détermination d'un signal utile et d'un signal parasite comprend les étapes suivantes :

- Estimation (120) d'un facteur multiplicatif (FM') entre le signal utile et le signal parasite ;
- Estimation (121) du signal utile à partir du facteur multiplicatif selon la relation : $DS'_n = (A_n+B_n) / (FM'+1)$, et $S'_n = Cumul (A+B)_n / (FM'+1)$, où $DS'_n$ est un niveau du signal utile, $S'_n$ est le signal utile, et $Cumul (A+B)_n$ est un niveau de signal accumulé au temps de $B_n$

et **en ce que** lequel l'étape (120) d'estimation du facteur multiplicatif (FM') entre le signal utile et le signal parasite comprend les étapes suivantes :

- à partir de 3 échantillons successifs de premiers signaux $(A_{n-1}, A_n, A_{n+1}) = (VA)_n$, estimation (122) d'une mesure $A'_n$ du signal parasite au temps du deuxième signal $B_n$ ;
- régression linéaire (123) sur 3 points successifs $\{(x_i,y_i) ; i = 1 \text{ à } 3\}$ pour obtenir un coefficient de proportionnalité FP, avec :

◦

$$\{x_i\} = [Cumul(A+B)_{n-2}, Cumul(A+B)_{n-1}, Cumul(A+B)_n]$$

◦

$$\{y_i\} = [Cumul(B-A')_{n-2}, Cumul(B-A')_{n-1}, Cumul(B-A')_n]$$

o $Cumul(A)_n$ étant égal à $A_0+A_1+...+A_n$

- Calcul (124) du facteur multiplicatif avec la relation $FM' = (FP)^{-1} - 1$.

2. Procédé de contrôle d'exposition selon la revendication 1, comprenant en outre une étape (104) d'adaptation de la dose de rayons X émise par le tube générateur (20) en fonction du signal utile transmis au bloc de contrôle (21) du tube générateur (20).

3. Procédé de contrôle d'exposition selon l'une quelconque des revendications 1 ou 2, dans lequel l'étape (103) de transmission du signal utile au bloc de contrôle du tube générateur est réalisée par transmission filaire.

4. Procédé de contrôle d'exposition selon l'une quelconque des revendications 1 ou 2, dans lequel l'étape (103) de transmission du signal utile au bloc de contrôle du tube générateur est réalisée par transmission sans fil, préférentiellement par transmission RF.

**Patentansprüche**

1. Verfahren zum Echtzeitsteuern von Bestrahlung mit einer Röntgenstrahlendosis, die von einer Röntgenstrahlgeneratorröhre (20) emittiert und von einem Detektor (10) empfangen wird, der einen Flachdetektor (11) umfasst, wobei die Generatorröhre (20) eine Steuereinheit (21) zum Steuern der Generatorröhre (20) umfasst, die zum Steuern einer von der Generatorröhre (20) emittierten Röntgenstrahlendosis konfiguriert ist, wobei der Flachdetektor (11) Folgendes umfasst:

   a. einen Satz von Pixeln (P(i,j)), die zu einer Matrix (13) nach Reihen (Li) und Spalten (Cj) organisiert und zum Erzeugen von Signalen auf der Basis der auf den Detektor (10) auftreffenden Röntgenstrahlendosis (22) konfiguriert sind;
   b. eine Schaltung (30), die zum Bestimmen eines Nutzsignals auf der Basis der Signale von mindestens einer der Reihen (Li) konfiguriert ist;
   c. ein Modul zum Übertragen (31) des Nutzsignals zur Steuereinheit zum Steuern der Generatorröhre (20);
   wobei das Steuerverfahren die folgenden Schritte umfasst:

      - Bestrahlen (100) des Flachdetektors (11) mit einer von der Röntgenstrahlgeneratorröhre (20) emittierten Röntgenstrahlendosis;
      - wiederholtes Auslesen (101) mindestens einer der Reihen (Li) von Pixeln (P(i,j)) während des Bestrahlens des Flachdetektors (11) mit der Röntgenstrahlendosis;
      - Bestimmen (102) eines Nutzsignals und eines Streusignals auf der Basis der Signale aus dem Auslesen der mindestens einen der Reihen (Li);
      - Übertragen (103) des Nutzsignals zu der Steuereinheit (21) zum Steuern der Generatorröhre (20);

   wobei das Steuerverfahren **dadurch gekennzeichnet ist, dass** der Schritt (101) des wiederholten Auslesens mindestens einer der Reihen (Li) von Pixeln für jede Reihe (Li) der mindestens einen der Reihen (Li) die folgenden Schritte umfasst:

      - Auslesen (110) der Spalte (Cj) ohne Aktivierung der Reihe Li, um ein erstes Signal An zu erhalten;
      - Auslesen (111) der Spalte (Cj) mit Aktivierung der Reihe Li, um ein zweites Signal Bn zu erhalten;

   dadurch, dass der Schritt (102) des Bestimmens eines Nutzsignals und eines Streusignals die folgenden Schritte umfasst:

      - Schätzen (120) eines Multiplikationsfaktors (FM') zwischen dem Nutzsignal und dem Streusignal;
      - Schätzen (121) des Nutzsignals auf der Basis des Multiplikationsfaktors gemäß der Beziehung:

   $DS'_n = (A_n+B_n) / (FM'+1)$, und $S'_n = Cumul (A+B)_n / (FM'+1)$, wobei $DS'_n$ ein Pegel des Nutzsignals ist, $S'_n$ das Nutzsignal ist, und $Cumul (A+B)_n$ ein über die Zeit akkumulierter Signalpegel von $B_n$ ist,
   und dadurch, dass der Schritt (120) des Schätzens des Multiplikationsfaktors (FM') zwischen dem Nutzsignal und dem Streusignal die folgenden Schritte umfasst:

      - Schätzen (122), auf der Basis von 3 aufeinanderfolgenden Abtastungen von ersten Signalen $(A_{n-1}, A_n, A_{n+1}) = (VA)_n$, eines Messwertes $A'_n$ des Streusignals zum Zeitpunkt des zweiten Signals $B_n$;
      - lineare Regression (123) an 3 aufeinanderfolgenden Punkten $\{(x_i, y_i); i = 1 \text{ bis } 3\}$, um einen Proportionalitätskoeffizienten FP zu erhalten, wobei:

         ∘

$$\{x_i\} = [Cumul(A+B)_{n-2}, Cumul(A+B)_{n-1}, Cumul(A+B)_n]$$

         ∘

$$\{y_i\} = [\text{Cumul}(B\text{-}A')_{n-2}, \text{Cumul}(B\text{-}A')_{n-1}, \text{Cumul}(B\text{-}A')_n]$$

◦ Cumul$(A)_n$ ist gleich $A_0+A_1+...+A_n$

- Berechnen (124) des Multiplikationsfaktors mit der Beziehung FM' = $(FP)^{-1}$-1.

2. Bestrahlungssteuerverfahren nach Anspruch 1, das ferner einen Schritt (104) des Anpassens der von der Generatorröhre (20) emittierten Röntgenstrahlendosis auf der Basis des zur Steuereinheit (21) zum Steuern der Generatorröhre (20) übertragenen Nutzsignals umfasst.

3. Bestrahlungssteuerverfahren nach Anspruch 1 oder 2, wobei der Schritt (103) des Übertragens des Nutzsignals zur Steuereinheit zum Steuern der Generatorröhre durch drahtgebundene Übertragung durchgeführt wird.

4. Bestrahlungssteuerverfahren nach Anspruch 1 oder 2, wobei der Schritt (103) des Übertragens des Nutzsignals zur Steuereinheit zum Steuern der Generatorröhre durch drahtlose Übertragung, vorzugsweise durch HF-Übertragung, durchgeführt wird.

**Claims**

1. A method for real-time control of exposure to an X-ray dose emitted by a generator tube (20) for generating an X-ray beam and received by a detector (10) comprising a flat panel detector (11), the generator tube (20) comprising a control unit (21) for controlling the generator tube (20) that is configured to control an X-ray dose emitted by the generator tube (20), said flat panel detector (11) comprising

   a. a set of pixels (P(i,j)) organised into a matrix (13) along rows (Li) and columns (Cj) and configured so as to generate signals on the basis of the X-ray dose (22) impinging on the detector (10);
   b. a circuit (30) configured to determine a payload signal based on the signals from at least one of the rows (Li);
   c. a transmission module (31) for transmitting the payload signal to the control unit for controlling the generator tube (20);
   said control method comprising the following steps:

      - exposing (100) the flat panel detector (11) to an X-ray dose emitted by the generator tube (20) for generating an X-ray beam;
      - repeatedly reading out (101) at least one of the rows (Li) of pixels (P(i,j)) while the flat panel detector (11) is exposed to the X-ray dose;
      - determining (102) a payload signal and a stray signal based on the signals from the readout of the at least one of the rows (Li);
      - transmitting (103) the payload signal to the control unit (21) for controlling the generator tube (20);

   said control method being **characterised in that** the step (101) of repeatedly reading out at least one of the rows (Li) of pixels comprises, for each row (Li) of the at least one of the rows (Li), the following steps:

      - reading out (110) the column (Cj) without activation of the row Li to obtain a first signal An;
      - reading out (111) the column (Cj) with activation of the row Li to obtain a second signal Bn;

   **in that** the step (102) of determining a payload signal and a stray signal comprises the following steps:

      - estimating (120) a multiplication factor (FM') between the payload signal and the stray signal;
      - estimating (121) the payload signal based on the multiplication factor using the relationship:
      DS'$_n$ = $(A_n+B_n)$/(FM'+1), and S'$_n$ = Cumul $(A+B)_n$/(FM'+1), where DS'$_n$ is a level of the payload signal, S'$_n$ is the payload signal, and Cumul$(A+B)_n$ is a signal level accumulated over time of B$_n$

   and **in that** the step (120) of estimating the multiplication factor (FM') between the payload signal and the stray signal comprises the following steps:

      - based on 3 successive samples of first signals $(A_{n-1}, A_n, A_{n+1})$ = $(VA)_n$, estimating (122) a measurement

A'$_n$ of the stray signal at the time of the second signal B$_n$;
- linear regression (123) on 3 successive points $\{(x_i,y_i); i = 1 \text{ to } 3\}$ to obtain a proportionality coefficient FP, where:

○

$$\{x_i\} = [Cumul(A+B)_{n-2}, Cumul(A+B)_{n-1}, Cumul(A+B)_n]$$

○

$$\{y_i\} = [Cumul(B-A')_{n-2}, Cumul(B-A')_{n-1}, Cumul(B-A')_n]$$

○ Cumul(A)$_n$ being equal to $A_0+A_1+...+A_n$

- calculating (124) the multiplication factor using the relationship FM' = $(FP)^{-1}-1$.

2. The method for the control of exposure according to claim 1, further comprising a step (104) of adapting the X-ray dose emitted by the generator tube (20) on the basis of the payload signal transmitted to the control unit (21) for controlling the generator tube (20).

3. The method for the control of exposure according to any one of claim 1 or 2, wherein the step (103) of transmitting the payload signal to the control unit for controlling the generator tube is performed through wired transmission.

4. The method for the control of exposure according to any one of claim 1 or 2, wherein the step (103) of transmitting the payload signal to the control unit for controlling the generator tube is performed through wireless transmission, preferably through RF transmission.

FIG. 1

FIG. 2

FIG. 3

FIG. 4

FIG. 5

FIG. 6

FIG. 7

**EP 3 984 461 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 5481053 B

- EP 1001665 A2